# EUROPEAN PATENT SPECIFICATION

(11) **EP 2 480 569 B1**
(45) Date of publication and mention of the grant of the patent: **13.04.2016**
(21) Application number: 10759588.6
(22) Date of filing: 23.09.2010
(51) Int. Cl.: C07K 14/505, C07K 1/16

(54) **PROCESS FOR THE PURIFICATION OF RECOMBINANT HUMAN ERYTHROPOIETIN (EPO)**
VERFAHREN ZUR REINIGUNG VON REKOMBINANTEM HUMANEM ERYTHROPOIETIN (EPO)
PROCÉDÉ DE PURIFICATION D'ÉRYTHROPOÏÉTINE HUMAINE RECOMBINANTE (EPO)

(30) Priority: 23.09.2009 EP 09012120
(43) Date of publication of application: 01.08.2012
(73) Proprietor: ratiopharm GmbH, 89079 Ulm (DE)
(72) Inventor: HINDERER, Walter, 63110 Rodgau (DE); ARNOLD, Stefan, 68723 Schwetzingen (DE)
(74) Representative: Müller Fottner Steinecke
(86) International application number: PCT/EP2010/005839
(87) International publication number: WO 2011/035914

(56) References cited:
- WO-A1-00/27869
- WO-A1-86/04068
- WO-A1-03/045996
- WO-A2-03/029291
- WO-A2-2010/036964
- US-A- 5 856 298
- US-A1- 2009 092 607
- US-A1- 2009 105 462
- SKIBELI VENKE ET AL: "Sugar profiling proves that human serum erythropoietin differs from recombinant human erythropoietin" BLOOD, AMERICAN SOCIETY OF HEMATOLOGY, US, vol. 98, no. 13, 15 December 2001 (2001-12-15), pages 3626-3634, XP002304285 ISSN: 0006-4971
- ELLIOT S ET AL: "Structural requirements for addition of O-linked carbohydrate to recombinant erythropoietin" BIOCHEMISTRY, AMERICAN CHEMICAL SOCIETY, EASTON, PA.; US, vol. 33, no. 37, 1 January 1994 (1994-01-01), pages 11237-11245, XP002237818 ISSN: 0006-2960
- DUBI S ET AL: "GLYCOSYLATION AT SPECIFIC SITES OF ERYTHROPOIETIN IS ESSENTIAL FOR BIOSYNTHESIS, SECRETION, AND BIOLOGICAL FUNCTION" JOURNAL OF BIOLOGICAL CHEMISTRY, AMERICAN SOCIETY OF BIOLOCHEMICAL BIOLOGISTS, BIRMINGHAM, US, vol. 263, no. 33, 25 November 1988 (1988-11-25), pages 17516-17521, XP002053702 ISSN: 0021-9258
- GOKANA A ET AL: "Chromatographic separation of recombinant human erythropoietin isoforms" JOURNAL OF CHROMATOGRAPHY, ELSEVIER SCIENCE PUBLISHERS B.V, NL, vol. 791, no. 1-2, 12 December 1997 (1997-12-12), pages 109-118, XP004107601 ISSN: 0021-9673 cited in the application
- HORTIN G L ET AL: "Lectin affinity chromatography of proteins bearing O-linked oligosaccharides: Application of jacalin-agarose", ANALYTICAL BIOCHEMISTRY, ACADEMIC PRESS INC, NEW YORK, vol. 188, no. 2, 1 August 1990 (1990-08-01), pages 271-277, XP024819659, ISSN: 0003-2697, DOI: 10.1016/0003-2697(90)90605-9 [retrieved on 1990-08-01]
- QUELLE F W ET AL: "HIGH-LEVEL EXPRESSION AND PURIFICATION OF A RECOMBINANT HUMAN ERYTHROPOIETIN PRODUCED USING A BACULOVIRUS VECTOR", BLOOD, AMERICAN SOCIETY OF HEMATOLOGY, US, vol. 74, no. 2, 1 August 1989 (1989-08-01) , pages 652-657, XP009039948, ISSN: 0006-4971
- MACMILLAN D ET AL: "SELECTIVE IN VITRO GLYCOSYLATION OF RECOMBINANT PROTEINS: SEMI-SYNTHESIS OF NOVEL HOMOGENEOUS GLYCOFORMS OF HUMAN ERYTHROPOIETIN", CHEMISTRY AND BIOLOGY, CURRENT BIOLOGY, LONDON, GB, vol. 8, no. 2, 1 January 2001 (2001-01-01) , pages 133-145, XP000998149, ISSN: 1074-5521, DOI: 10.1016/S1074-5521(00)90065-6
- STORRING P L ET AL: "Epoetin alfa and beta differ in their erythropoietin isoform compositions and biological properties", BRITISH JOURNAL OF HAEMATOLOGY, WILEY-BLACKWELL PUBLISHING LTD, GB, vol. 100, 1 January 1998 (1998-01-01), pages 79-89, XP003000623, ISSN: 0007-1048, DOI: 10.1046/J.1365-2141.1998.00521.X
- NAGANO M ET AL: "Detection of isoforms of recombinant human erythropoietin by various plant lectins after isoelectric focusing", ELECTROPHORESIS, WILEY INTERSCIENCE, DE, vol. 26, 1 January 2005 (2005-01-01), pages 1633-1645, XP003023867, ISSN: 0173-0835, DOI: 10.1002/ALPS.200410214
- BROUDY V C ET AL: "Recombinant human erythropoietin: Purification and analysis of carbohydrate linkage", ARCHIVES OF BIOCHEMISTRY AND BIOPHYSICS, ACADEMIC PRESS, US, vol. 265, no. 2, 1 September 1988 (1988-09-01), pages 329-336, XP024761869, ISSN: 0003-9861, DOI: 10.1016/0003-9861(88)90135-X [retrieved on 1988-09-01]
- KODAMA DAISUKE ET AL: "Production of human erythropoietin by chimeric chickens", BIOCHEMICAL AND BIOPHYSICAL RESEARCH COMMUNICATIONS, vol. 367, no. 4, March 2008 (2008-03), pages 834-839, ISSN: 0006-291X
- YANG M ET AL: "Effects of ammonia on CHO cell growth, erythropoietin production, and glycosylation.", BIOTECHNOLOGY AND BIOENGINEERING 20 MAY 2000, vol. 68, no. 4, 20 May 2000 (2000-05-20), pages 370-380, ISSN: 0006-3592
- ZOU Z ET AL: "[Purification of recombinant erythropoietin by reversed-phase high performance liquid chromatography (RP-HPLC)].", SE PU = CHINESE JOURNAL OF CHROMATOGRAPHY / ZHONGGUO HUA XUE HUI MAY 1998, vol. 16, no. 3, May 1998 (1998-05), pages 263-264, ISSN: 1000-8713

## Description

### Field of the Invention

The present invention relates to a procedure for the production of erythropoietin (EPO), in particular recombinant human EPO (rhEPO) with a defined composition of glycoforms in a highly pure form, i.e. with a high amount of O-glycosylated EPO isoforms. This is achieved by using a specific combination of chromatographic steps.

### Background to the Invention

Erythropoietin is the principal hormone regulating the proliferation and differentiation of erythroid progenitor cells and the maintenance of physiological levels of circulating red blood cells. In the fetus EPO is primarily produced in the liver and about 90% of its production switches to the kidney after birth. When EPO levels fall due to chronic or acute renal failure, EPO must be externally administered to prevent a rising anemia. A therapeutically active human erythropoietin has been available since the discovery of the EPO gene and its expression in rodent cells. The native human erythropoietin is encoded by a gene on chromosome 7 at 7q11-q22 (Law et al., Proc. Natl. Acad. Sci. USA 83 (1986), 6920-6924). Recombinant human EPO has entered the market in 1989 when the FDA gave clearance for its use in the treatment of anemia associated with chronic renal failure. As this is the case also for other recombinant glycoproteins that are used as drugs, the glycosylation pattern of EPO has a prominent influence on the bioavailability, on the bioactivity, and on immunogenic properties of the glycoprotein. For the production of rhEPO, Chinese hamster ovary (CHO) and baby hamster kidney (BHK-21) host cells play an important role as expression systems, and therefore have been thoroughly studied (Sasaki et al., J. Biol. Chem. 262 (1987), 12059-12076; Takeuchi et al., J. Biol. Chem. 263 (1988), 3657-36633; Nimtz et al., Eur. J. Biochem. 213 (1993), 39-56; Tsuda et al., Biochemistry 27 (1988), 5646-5654). It is well established that these cell lines produce glycoforms that most closely resemble human glycoforms. It is also well known that carbohydrates play a major role in glycoprotein targeting and clearance (Drickamer et al., Annu. Rev. Cell. Biol. 9 (1003), 237-264; Helenius et al., Science 291 (2001), 2364-2369). Lately, a novel form of erythropoietin with two extra N-glycosylation sites, called darbepoetin alfa (ARANESP®), had been approved by the EMEA and by the FDA.

The human EPO gene encodes a 27 amino acid signal peptide and a 166 amino acid protein with a calculated molecular weight of 18396 Dalton. The mature protein usually has a single amino acid N-terminal deletion and is 165 amino acids in length. The signal sequence directs the peptide to the cellular compartments involved in the proper glycosylation, leading to a mature protein with three N- and one O-glycan. The sugar moieties, which make about 40% of the total molecular weight, are essential for the full biological activity of EPO in vivo. Several studies have shown that the number of terminal sialic acid residues have an positive effect on the in vivo half-life, although the in vitro activity, i.e. the binding to the receptor, is highest in the non or partly glycosylated form (Takeuchi and Kobata, Glycobiology 1 (1991), 337-346). The degree on sialylation is directly proportional to the half-life, where those isoforms with less sialic acids are much faster cleared from the organism and therefore show low activity. In this context, Rush et al. (Anal. Chem. 67 (1995), 1442-1452) described O-acetylation of sialic acid residues of erythropoietin and their effect on increasing circulation time indicating that a high degree of O-acetylation of sialic acid residues increases circulation time by reducing hepatic clearance.

Typically, EPO preparations obtained by recombinant expression in mammalian cells, especially in CHO cells contain up to 50% of EPO isoforms which lack the O-glycan and thus loose the potential for two more sialic acid residues per molecule. Therefore, it would be desirable to provide an EPO preparation which is substantially free of such deficient, non-O-glycosylated isoforms and methods for providing same.

Methods of isolation/purification of EPO which are known from the scientific and patent literature comprise different chromatographic steps. The most commonly used are anion exchange chromatography (AEX) and reverse phase HPLC (RP-HPLC). Other chromatographic methods are also used, for example hydroxyapatite, hydrophobic interaction (HIC), cation exchange (CEX), affinity (i.e. immunoaffinity or dye ligands) and size exclusion (gel filtration) (SEC) chromatography. Some intermediate steps are also common such as concentration, diafiltration, ultrafiltration, dialysis, precipitation with ethanol, salt and others.

EP 205 564 describes the purification of EPO using a cation exchange chromatographic step followed by subsequent RP-HPLC.

EP 228 452 and US 4,667,016 described EPO purification by an anion exchanger followed by RP chromatography and gel chromatography.

EP 428 267 describes an improvement of the method according to EP 228 452 dealing with the isolation of (particularly acidic) isoforms by means of a further anion exchanger after RP chromatography.

EP 1 394 179 describes a purification process comprising dye affinity chromatography, HIC, hydroxyapatite, RP-HPLC and an anion exchanger.

EP 1 428 878 describes a method of purifying highly sialylated EPO isoforms by using a first anion exchange chromatography step as a capture step and an optional acidic wash step, hydrophobic interaction chromatography, affinity chromatography, and a second anion exchange chromatography step with an acidic wash step.

WO99/28346 describes the purification of EPO to a high degree on N-acetyl-lactosamine units and/or tetra-antennary branches in the carbohydrate structure. The purification process starts with a cell culture supernatant, capture by affinity chromatography, purification by hydrophobic interaction chromatography, hydroxyapatite and reverse phase chromatography. WO03/045996 describes a method for recovering and purifying rhEPO from a cell culture medium comprising inter alia the steps of anion exchange chromatography, reverse phase chromatography, anion exchange chromatography and size exclusion chromatography.

WO 03/080852 described a process for the production of EPO including at least dye affinity chromatography, hydrophobic chromatography and anion exchange chromatography, optionally further including gel filtration chromatography.

Miyake et al., J. Biol. Chem 252 (1977), 5558-5564 describe the purification of urinary EPO by a seven step procedure, including ion exchange chromatography, ethanol precipitation, gel filtration and adsorption chromatography.

Broudy et al., Arch. Biochem. Biophys. 265 (1988), 329-336 used a transfected BHK cell line to purify EPO by Affi-Gel Blue chromatography, anion exchange chromatography and reverse phase chromatography.

Gokana et al., J. Chromatography 791 (1997), 109-118 describe the separation of recombinant human EPO isoforms by means of DEAE chromatography, subsequent to previous purification of the EPO by immunoaffinity. The separation of the EPO isoforms is based on their different pIs.

Goto et al., Bio/Technology 6 (1988), 67-71 describe purification of EPO by means of immunoaffinity, gel chromatography and hydroxyapatite.

Holcke et al., Eur. J. Biochem. 228 (1995), 981-1008 describe glycan analysis of EPO including PNGase treatment for separate analysis of N-glycans and O-glycans.

Kishino and Miyazaki, J. Chromatography 699 (1997), 371-381 review methods for glycoprotein analysis including purification of EPO from urine using terminal RP chromatography.

Nimtz et al., Eur. J. Biochem. 213 (1993), 39-56 describe analysis of EPO glycosylation including PNGase F digestion, showing that recombinant human EPO expressed in and obtained from baby hamster kidney (BHK) cells is only 60% O-glycosylated.

Quelle et al., Blood 74 (1989), 652-657 describe the purification of EPO from insect cells, wherein the purification process comprises anion exchange chromatography and RP-HPLC and subsequent lectin affinity chromatography.

Sasaki et al., J. Biol. Chem. 262 (1987), 12059-12076 and Sasaki et al., Biochemistry 27 (1988) 8618-8626 describe analysis of the carbohydrate structure of recombinant human EPO expressed in and obtained from Chinese hamster ovary (CHO) cells. EPO was purified by means of RP-HPLC, among other methods.

Skibell et al., Blood 98 (2001), 3626-3634 describe the examination of the glycan structure of EPO from human serum compared to recombinant EPO demonstrating that O-glycosylation also occurs with EPO circulating in the blood.

Sytkowski and Donahue J. Biol. Chem. 262 (1987), 1161-1166 determined the EPO receptor binding site determined (indirectly by means of neutralization) with the aid of monoclonal antibodies (mAb). Table II shows that a mAb directed against the EPO amino acid sequence 111-129 displays the strongest neutralization effect indicating that the O-glycan located at Ser126 might be involved in receptor binding.

US 2009/105462 A1, Dubé et al. J. Biol. Chem. (1988), 17516-17521, WO 03/029291 A2, Hortin et al. Anal. Biochem. (1990), 271-277 and Macmillan et al. Chem. Biol. (2001), 133-145 relate to glycosylated EPO.

Elliot et al. Biochemistry (1994), 11237-11245 and US 2009/092607 A1 describe EPO variants and Fc-EPO fusion proteins, respectively.

WO 00/27869 A1 and Zou et al. (1998) describe EPO purification methods.

Storring et al. Br. J. Haematol. (1998), 79-89, Nagano et al. Electrophoresis (2005), 1633-1645 and US 5,856,298 relate to EPO isoforms.

WO 86/04068 A1 describes homogenous EPO.

Hence, while the significance of glycosylation of EPO was known, none of the mentioned documents discloses a method for providing an EPO preparation which is (i) substantially free of non O-glycosylated isoforms, (ii) purified to a pharmaceutical grade, (iii) free of viral contaminants, and (iv) amenable to large scale production. This technical problem is solved by the embodiments as characterized in the claims and described further below.

### Summary of the Invention

The present invention provides a method for recovering and purifying human erythropoietin (rhEPO) from a cell culture medium derived from recombinant cells, which method comprises a reverse phase (RP)-HPLC chromatography step, and a subsequent cation exchange (CEX) chromatography step, wherein the RP-HPLC step is preceded by an anion exchange (AEX) chromatography step. In particular, the present invention relates to a method of purifying biologically active O-glycosylated at serine 126 erythropoietin (EPO) isoforms from a complex protein mixture in pure form suitable as a drug substance, wherein the method comprises:
(a) a dye affinity chromatography step for capturing and concentrating the EPO containing solution and providing the major reduction of potential contaminants;
(b) an anion exchange chromatography step for the enrichment of acidic isoforms of EPO, further removal of contaminants and the elimination of any dye ligand that may have leached from the first column;
(c) a reverse phase high performance liquid chromatography (RP-HPLC) step under conditions in order to remove EPO molecules that are not glycosylated at the Ser126 residue and to remove contaminants;
(d) a cation exchange chromatography step for removing RP-HPLC solvents and aggregated species, to exchange the buffer, and to concentrate the EPO fraction; and
(e) a size exclusion chromatography step as the final chromatography step employed to remove any possible remaining aggregates and other contaminants.

Recombinant human erythropoietin (rhEPO) purified in accordance with the method of the present invention has been subjected to several analytical procedures in order to characterize the N- and O-glycosylation status of the EPO. As mentioned in the background section, EPO has one O-glycosylation site at S126, and three N-glycosylation sites at N24, N38, and N83. The overall glycosylation pattern of the EPO preparation of the present invention was found to be rather similar to the one found in the international BRP standard, and in a commercial reference product. For the O-linked oligosaccharides, however, it was observed that in comparison to the BRP standard the relative amount of non-glycosylated forms of EPO was significantly lower, if present at all. This may lead to an improved EPO preparation of the present invention as compared to other commercial products.

Without intending to be bound by theory it is believed that the virtually selective separation of the O-deglycosylated (Des-O) EPO forms in the HPLC functions due to the fact that the entire O-sugar chain is lacking. In case of the N-glycans the differences are not as pronounced. Here, only the occasional antenna is missing, but rarely, if ever, the entire glycan. Isoforms lacking N-glycans (minus four sialic acids per glycan) would already be strongly depleted in the anion exchange chromatography (AEX) step. In case the O-chain is missing a hydrophobic patch (appr. aa121-130) is exposed, which is normally shielded by the sugar chain. Indeed, upon closer inspection of the O-glycosylation site, a dense accumulation of hydrophobic amino acids surrounding the Serin126 can be discerned. Four alanines directly surround the Serin126 and three prolines (121, 122, 129) define a conspicuously hydrophobic area. In addition, the Leucine130 is also hydrophobic and contributes to this effect. Said additional hydrophobic site in EPO enhances the overall binding force to the C4 matrix of the HPLC column. Thus, the Des-O forms eluate at a later point in time.

In the present invention a five column chromatographic process is provided for purification of EPO in pure form suitable as a drug substance, which process comprises:
(a) Dye affinity chromatography for capturing and concentrating the EPO containing solution and providing the major reduction of potential contaminants;
(b) Anion exchange chromatography for the enrichment of acidic isoforms of EPO, further removal of contaminants (e.g. DNA, HCP) and the elimination of any dye ligand that may have leached from the first column;
(c) RP-HPLC under conditions in order to remove EPO molecules that are not glycosylated at the Ser126 residue and to remove contaminants;
(d) Cation exchange chromatography for removing RP-HPLC solvents and aggregated species, to exchange the buffer, and to concentrate the EPO fraction; and
(e) Size exclusion chromatography as the final chromatography step employed to remove any possible remaining aggregates and other contaminants.

Furthermore, two specific virus removal/inactivation steps are included in the EPO manufacturing process. First, following the RP-HPLC step, the eluate is held in the acidic acetonitrile:water plus 0.1% (v/v) TFA mixture for 60 to 180 minutes at 22 ± 3°C. Second, a nanofiltration step is included following the final chromatography step to increase the virus safety of the drug substance. In addition, the anion exchange chromatography has been demonstrated to be an effective step for removal of virus too. The resultant EPO drug substance can be dispensed, using for example a peristaltic pump, into bulk drug substance storage containers of 250 ml or 30 ml volumes and can be stored at ≤ -70°C.

Therefore, the present method provides a profile-directed production of EPO and highly uniform product of high quality. Furthermore, the present invention enables large scale preparation of high quantities of biologically active EPO with high purity and a desired profile of O-glycosylated EPO isoforms.

The EPO purity is high by reaching a purity exceeding at least 99 % of total proteins and advantageously exceeding 99.9 % of total proteins, as determined by HPLC and gel electrophoresis. Furthermore, the risk of contamination by viruses and the like and hence the clinical safety of the product is improved by lowering the risk of infections. In this context, an advantageous side effect of using RP-HPLC and an organic solvent for eluting and storing the EPO sample in between proceeding with the next step in the purification process resides in the inactivation of viruses which otherwise may remain viable in other solvents used for example in hydrophobic interaction or ion exchange chromatography.

In this context, the person skilled in the art will acknowledge that one essential feature of the present invention consists of the performance of RP-HPLC and a subsequent CEX chromatography step while any of the other chromatographic steps may be altered or omitted at all. This may also apply to the AEX step which, though preferably incorporated in the method of the present invention, may be replaced by a different purification step or may also be omitted depending on the EPO sample applied and/or in case it is desired to provide a heterologous or low-sialylated while O-glycosylated EPO preparation, for example.

The fact that O-glycosylation of the EPO isolated in accordance with the method of the present invention was more complete than for the BRP standard indicates that the half life of the EPO preparation may be well improved. Therefore, it is prudent to expect that *the in vivo* half life of the EPO preparation of the present invention is at least similar if not improved to the recombinant EPO products hitherto commercially available. This also implies that pharmacokinetic and pharmacodynamic properties otherwise will be similar to the ones of market products. The EPO obtained in accordance with the method of the present invention is thus particularly suitable for use in human medicine.

### Brief description of the Drawings

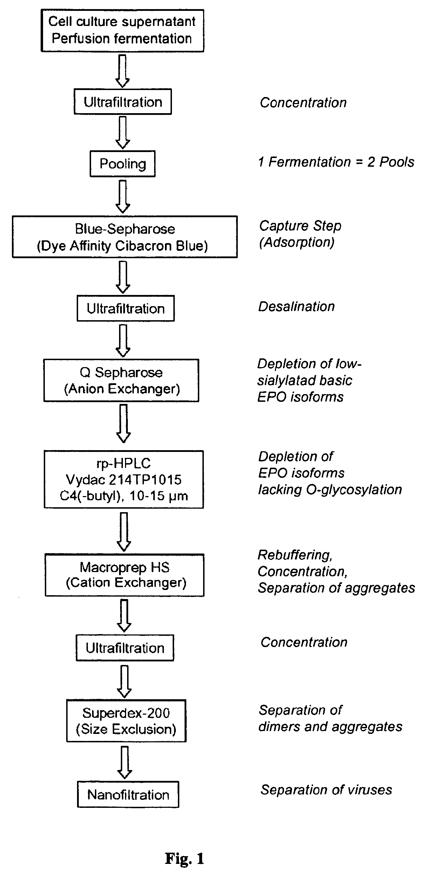

**Fig. 1****:** Scheme of purification procedure for the isolation of a specific mixture of highly sialylated and O-glycosylated EPO isoforms. The significance and intended purpose of the individual purification steps is further explained in the description.

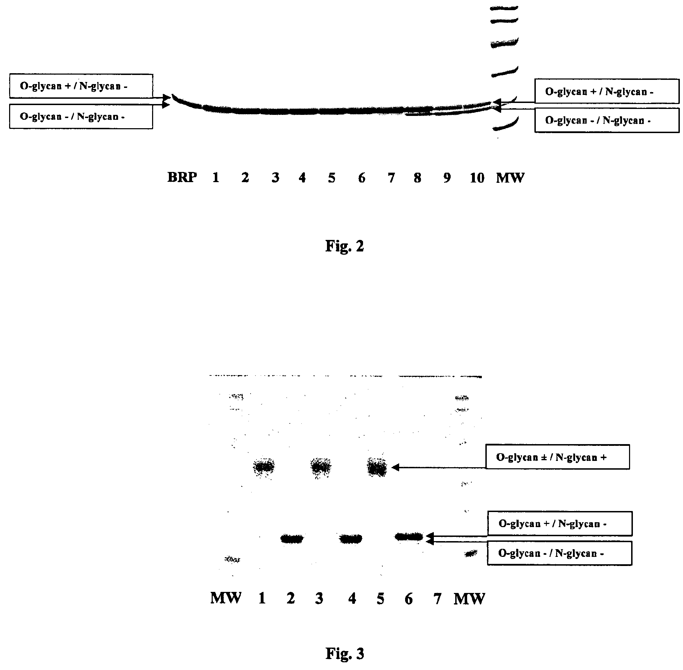

**Fig. 2****:** Photograph of a Coomassie-stained 12.5% SDS-PAGE gel of HPLC fractions after the digestion with PNGase. 5 µg protein were loaded per slot. MW, molecular weight marker; BRP, BRP standard batch 1 (= biological reference preparation = a mixture of epoetin alpha and beta); 1 = EPO sample after anion exchange chromatography (AEX pool) and prior to subjecting to the RP-HPLC step; 2 - 10 = samples of EPO eluate fractions obtained after subjecting the AEX pool to the RP-HPLC and application of linear gradient of solvent as described in the Examples; see also table 6. Each EPO batch is shown after digestion with polypeptide N-glycosidase (PNGase) which removes all N-glycans, but leaves the O-glycan attached, if present. The lower, thinner band is the non-O-glycosylated (Des-O) form. The enzyme itself may also be detected in the gel as a trace band in the middle of the gel. The left lane shows the BRP standard, the lane next to it shows the column application (contains the Des-O form, like BRP standard), followed by nine fractions of the gradient elution. It can be seen that the first five fractions are apparently free of the Des-O form, which is mainly present in the last three to four fractions confirming that the Des-O isoform remained bound at the chromatographic material and eluate from the column at a later point in time. The pool criteria regulate the remaining proportion of the Des-O isoform.
**Fig. 3****:** Photograph of a Coomassie-stained 12.5% SDS-PAGE gel. MW, molecular weight marker; 1 - 4 = EPO preparations obtained by the method as illustrated in the Examples; 5 + 6 = BRP standard batch 1 (= biological reference preparation = a mixture of epoetin alpha and beta); 7 = blank. Each EPO batch is shown before (1, 3, 5) and after digestion (2, 4, 6) with PNGase. The enzyme itself may also be detected in the gel as a trace band. The BRP standard exhibits a double band after digestion. The lower, thinner band is the non-O-glycosylated (Des-O) form. It can be seen that the EPO preparation obtained according to the method of the present invention are apparently lacking the lower band, confirming that the Des-O form has been removed during the HPLC step.

### Detailed Description of the Invention

The present invention provides a method for the isolation and purification of improved EPO preparations with only minor contents of non-O-glycosylated isoforms, if any, and substantially free of aggregates. This object is achieved by diminishing the content of non-O-glycosylated EPO isoforms through reverse phase (RP) - high pressure liquid chromatography (HPLC) under appropriate conditions and the subsequent application of a cation exchange chromatography (CEX) step. More specifically, the present invention relates to a method of purifying glycosylated erythropoietin (EPO) isoforms from a complex protein mixture as characterized in the claims, wherein the method comprises an anion exchange (AEX) chromatography step and a cation exchange (CEX) chromatography step, which are separated by a reverse phase (RP) chromatography step.

The term "isoform", as used herein, refers to a glycoprotein preparation/fraction that contains glycoproteins which have identical amino acid sequence but distinct isoelectric points as revealed for example by Isoelectric Focussing (IEF) gels or distinct number of charges as revealed for example by Capillary Zone Electrophoresis (CZE). These differences reflect the hetereogeneity in the glycosylation pattern. Individual EPO molecules may differ in respect to the extent, to the complexity, to the nature, to the antennarity and to the order of attached glycosyl-, sialyl-, and acetyl groups. Even charged anorganic groups like phosphate and sulphate, may contribute to the nature of a specific isoform. Thus, glycoprotein isoforms according to the invention are defined by their distinct isoelectric point and their identical amino acid sequence and each isoform may therefore actually comprise multiple different EPO molecules in the strict chemical sense.

In accordance with the method of the present invention the anion exchange chromatography is preferably used to select EPO isoforms, in particular highly sialylated acidic EPO isoforms; see also Fig. 1. According to the present invention "acidic isoforms" of EPO comprise those isoforms that have a high degree or content of glycosyl-, and preferably of sialyl-groups, thus appearing with a low (acid) pI. Recombinant human EPO, when analyzed from cell culture supernatant by IEF, shows a broad isoelectric point (pI) isoform profile with up to maximum 14 different isoforms over a range of pI 3-9 (Gokana et al., J. Chromatogr. 791 (1997), 109-118). The EPO isoforms arise mainly due to their variant glycosyl content with variant numbers of negatively charged terminal sialic acid residues. EPO forms with more sialic acid residues and thus a more acidic pI are known to be of higher biological activity and therapeutic value because the terminal sialic acid residues on glyco-structures prevent the EPO from rapid clearance *in vivo* via the asialo-receptor route.

The anion exchange chromatography step may be performed with anion exchange resins or membranes that contain Diethylaminoethyl-groups (DEAE), quaternary aminoethyl-groups (QAE), quaternary ammonium-groups (Q), Dimethylaminoethyl-groups (DMAE) and/or Trimethylaminoethyl-groups (TMAE) as functional groups. Exemplary anion exchange materials are Dowex.RTM. 1 available from Dow chemical company, AG.RTM. (e.g., type 1, 2, 4), Bio-Rex.RTM. 5, DEAE Bio-Gel 1, Macro-Prep.RTM. DEAE all available from BioRad Laboratories, anion exchange resin type 1 available from Eichrom Technologies Inc., Source Q, ANX Sepharose 4, DEAE Sepharose (e.g., type CL-6B, FF), Q Sepharose, Capto Q, Capto S all available from GE Healthcare, AX-300 available from PerkinElmer, Asahipak ES-502C, AXpak WA (e.g., type 624, G), IEC DEAE all available from Shoko America Inc., Amberlite.RTM. IRA-96, Toyopearl.RTM. DEAE, TSKgel DEAE all available from Tosoh Bioscience GmbH, Mustang Q available from Pall Corporation. In a preferred embodiment of the method of the present invention the anion exchange chromatography step is performed with Q-Sepharose; see also the Examples. As described, the depletion of low sialylated basic and selection of highly sialylated acidic EPO isoforms, respectively, is preferably performed with a linear salt gradient from 0 to 200mM NaCl in a buffer comprising 20mM Tris-HCl at a pH of about 7,0.

Furthermore, in accordance with the method of the present invention reverse phase chromatography step is used to select O-glycosylated EPO isoforms. As shown in the Examples, such EPO isoforms may be eluted with a linear gradient of an organic solvent, preferably from 0 to 70% acetonitrile in water and containing about 0.1% TFA. In addition, or alternatively, an isocratic elution of EPO with a solvent containing acetonitrile and about 0.1% TFA in water is used. Means and methods for performing reverse phase (RP) chromatography are well known to the person skilled in the art; see also the prior art recited in the background section, supra. Preferably, the RP chromatography step comprises reverse phase high performance liquid chromatography (RP-HPLC). Typically, the RP-HPLC is performed with resins that contain Methyl-, Butyl-, Phenyl-, Propyl- and/or Octyl- groups as functional groups. In a preferred embodiment of the method of the present invention the RP-HPLC is performed with a commercially available C4 reverse phase chromatography material. Exemplary reverse phase materials are: Vydac 214TPB1015, C4 available from Grace Davison; Daisopak SP-300-15-C4-BIO available from DAISO Fine Chem. GmbH;YMC Gel Butyl Sphärisch C4, 15µ, 300A available from YMC Europe GmbH; Jupiter 15µ, C4, 300A available from Phenomenex.
Most preferably, Vydac C4 (Vydac) is used consisting of silica gel particles, the surfaces of which carry C4-alkyl chains. The separation of EPO from the proteinaceous impurities is based on differences in the strength of hydrophobic interactions. Elution is performed with an acetonitrile gradient in water in presence of diluted trifluoroacetic acid. Usually, part of the "EPO peak" has to be cut off by fractionation. For example, in a number of about 9 or 10 eluate fractions containing EPO the last one to four samples which typically can make up to 40% of the entire EPO peak, have to be discarded while the remaining eluate pool is further purified in the subsequent steps; see also Figure 2.

In accordance with the present invention preparative RP-HPLC is usually performed at high pressure > 10 bar (on a preparative scale up to 30 - 40 bar) and small beads (5 - 10 µm), usually silica gel, which leads to better resolution. Preferably, the pH of the solvents, acidified with TFA, is about 2. Application of the AEX eluate at low acetonitrile concentration (e.g. 10% or pure water) and increasing acetonitrile concentrations gradient will elute the EPO isoforms.

In this context, it is observed that RP-HPLC as used in accordance with the method of the present invention is substantially different from common RP chromatography (RPC) which is performed at low pressure (also referred to as medium pressure method, < 10 bar, usually 3-5 bar). For example, RPC is typically performed with 15 µ beads or 30 µ beads as described in international application WO03/045996 (RP-Source 30). Furthermore, though like in HPLC organic solvents can be utilized, the RPC step in WO03/045996 begins subsequently to an ammonium sulfate precipitation with 0.24 M ammonium sulfate in the sample, which would not be favorable for HPLC but very suitable for HIC (hydrophobic interaction chromatography), where typically a decreasing salt gradient is applied for elution. Hence, the RPC as described in international application WO03/045996 is conducted in a fully aqueous solvent system, i.e. Tris / HCl buffer at pH 7 because hitherto no appropriate method was described for rapidly separating the organic solvent and for avoiding forming/dragging aggregates within the EPO preparation. However, the dispensation of an HPLC step with the use of an organic solvent is done at the expense of a less efficient separation of EPO isoforms, for example due to the larger bead particles used in RPC and the less stringent separation conditions. At any rate, the separation performance of an RP-HPLC is superior to that of an RPC.

A further important factor for the effectiveness of the purification scheme is that a cation exchange chromatography (CEX) is carried out in the third step, immediately after HPLC. This step is novel regarding the mode of application. After the RP chromatography, the EPO must be quickly subjected to buffer exchange as it is not stable in the acidic acetonitrile, i.e. there is a gradual formation of aggregates with time and increasing temperature. Moreover, for a subsequent gel chromatography used as final polishing step, the sample volume must be very small, i.e. the concentration of EPO in the RP pool has to be strongly increased. On the other hand the RP-HPLC pool has a rather large volume due to the relatively flat gradient required for separation of the Des-O-form. In accordance with the present invention a CEX chromatography step with a selected material (Macroprep S) has been developed instead of using a standard ultra/diafiltration step, which is a common but quite time-consuming method. This CEX chromatography allows particularly high flow rates and tolerates acidic acetonitrile in high concentrations. Due to the fact that it is positively charged in an acidic environment, EPO is a strong binder and is eluted with a very steep gradient, in a small volume and at a high concentration in the desired buffer. Surprisingly, the CEX chromatography also resulted in the separation of EPO aggregates, which inevitably form in acetonitrile, since it turned out that EPO aggregates do not elute but remain on the column and subsequently elute with the CIP solution in the regenerate. Thus, in accordance with the present invention the cation exchange chromatography step is used after RP chromatography, in particular RP-HPLC for buffer exchange, concentration of EPO and elimination of EPO aggregates; see also Fig. 1.

Different types of cation exchange materials are also available under different names and from a multitude of companies such as Bio-Rex.RTM. (e.g., type 70), Chelex.RTM. (e.g., type 100), Macro-Prep.RTM. (e.g., type CM, High S, 25 S), AG.RTM. (e.g., type 50W, MP) all available from BioRad Laboratories; WCX 2 available from Ciphergen, Dowex.RTM. MAC-3 available from Dow chemical company, Mustang C and Mustang S available from Pall Corporation, Cellulose CM (e.g., type 23, 52), hyper-D, PartiSphere available from Whatman plc., Amberlite.RTM. IRC (e.g., type 76, 747, 748), Amberlite.RTM. GT 73, Toyopearl.RTM. (e.g., type SP, CM, 650M) all available from Tosoh Bioscience GmbH, CM 1500 and CM 3000 available from BioChrom Labs, SP-Sepharose.TM., CM-Sepharose.TM. available from GE Healthcare, Porous resins available from PerSeptive Biosystems, Asahipak ES (e.g., type 502C), CXpak P, IEC CM (e.g., type 825, 2825, 5025, LG), IEC SP (e.g., type 420N, 825), IEC QA (e.g., type LG, 825) available from Shoko America Inc., 50W cation exchange resin available from Eichrom Technologies Inc. Preferably the cation exchange material is a strong cation exchange material such as Macro-Prep.RTM. High S or 25S, MacroCap SP, Toyopearl.RTM. SP 650M, Source S, SP Sepharose, or POLYCAT A. In one embodiment the cation exchange material is a sulfopropyl cation exchange material. In a preferred embodiment of the method of the present invention the cation exchange chromatography step is performed with Macro-Prep High S; see also the Examples.

In the method of the present invention the above described chromatographic steps are preceded by an affinity chromatography step as a capture step. Usually, the affinity chromatography step is performed with a dye chromatography resin, for example with commercially available Blue-Sepharose; see also the Examples. Preferably, the chromatographic steps in the method of the present invention are performed in the following order:
(a) an affinity chromatography step as a capture step;
(b) an anion exchange chromatography step;
(c) a reverse phase (RP) chromatography step; and
(d) a cation exchange chromatography step; see also Fig. 1 and the Examples.

In the first step the dye chromatography mainly removes contamination by proteases. A blue triazine dye such as Cibachron® blue is preferably used as the dye. Other triazine dyes are also suitable. The support material for the dye chromatography is not critical, however, a support material based on polysaccharides is preferably used such as e.g. Sepharose, preferably Sepharose 6 Fast Flow. The enrichment of highly sialylated and O-glycosylated EPO isoforms in accordance with the present invention is carried out in the subsequent chromatographic steps; see also Fig. 1.

In a preferred embodiment of the method of the present invention the elution of EPO in one or more of the chromatography steps is carried out by a step or gradient elution. The terms "step elution" and "step elution method", which are used interchangeably within this application, denote a method wherein the concentration of a substance causing elution, i.e., the dissolution of a bound compound from a material, is raised or lowered at once, i.e., directly from one value/level to the next value/level. In this "step elution" one or more conditions, for example the pH, the ionic strength, concentration of a salt, concentration of an organic compound, and/or the flow of a chromatography, is/are changed all at once from a first, e.g., starting, value to a second, e.g., final, value. This means, the conditions are changed incrementally, i.e. stepwise, in contrast to a steadily linear or non-linear change. In the "step elution method" a new fraction is collected after each increase in the ionic strength or content organic solvent. This fraction contains the compounds recovered from the ion exchange material with a corresponding increase in ionic strength and hydrophobicity, respectively. After each increase the conditions are maintained until the next step in the elution method is carried out. Typically, in large scale productions, whenever possible, gradient elutions are displaced by step or isocratic elutions.

In this context, any of the chromatographic steps performed in accordance with the method of the present invention may be performed either in a gradient or isocratic manner; see for review, e.g., Schellinger and Carr, J. Chromatography. 1109 (2006), 253-266. In particular, for the RP-HPLC and the anion chromatographic (AEX) step it is envisaged to replace the linear gradient with an isocratic elution. Accordingly, in one embodiment of the method of the present invention the RP and/or the AEX step are performed by isocratic elution.

In a further chromatographic step of the method of the present invention the EPO preparation obtained from cation exchange chromatographic (CEX) is polished by size exclusion chromatography (SEC), which removes potential dimers, higher aggregates and undesired small molecules such as process-related impurities, and also performs a buffer exchange for the final formulation, if appropriate. Typically, the size exclusion chromatography step is performed with a gel-filtration medium selected from the group of Superdex, Sephacryl, Sephadex, Sepharose, Fractogel, Toyopearl and Bio-Gel. Preferably, the size exclusion chromatography step is performed with commercially available Superdex-S200; see also the Examples.

In order to achieve a higher product concentration of the EPO preparation obtained after the CEX chromatography the eluate is preferably further concentrated before the gel-filtration (SEC). This is usually performed by an ultrafiltration step using a 5 - 10 kDa UF membrane leading to an about 10 fold concentrated UF-retentate with approx. 5 to 20 mg EPO per ml to give the SEC pool; see also the Examples.

To remove a potential virus load an additional dead-end virus-filtration step is implemented in the method of the present invention. This filtration is performed with a special membrane, designed to remove particles as small as 15 nm, such as the Planova 15N (Asahi). Alternative dead-end nanofiltration units are PALL Ultipor VF Grade DV20 or Millipore Viresolve NFP cartridges or capsules. Especially for small non-enveloped viruses (e.g. parvovirus), there is almost no other tool of virus removal or inactivation. The sterile filtered SEC-pool is passed over a dead-end filter with a suitable membrane and the filtrate represents the final bulk drug substance. Alternatively the nanofiltration can be inserted between the UF-concentration and the size exclusion chromatography. Hence, the method of the present invention may comprise prior to one or more of the chromatography steps an ultrafiltration step, and optionally a nanofiltration step, the latter preferably as the final step; see also Fig. 1.

In a particular preferred embodiment the method of the present invention comprises the following steps
(a) an affinity chromatography step as a capture step;
(b) an anion exchange chromatography step;
(c) a reverse phase (RP) chromatography step;
(d) a cation exchange chromatography step;
(e) a size exclusion chromatography step;
(f) a nanofiltration step; and
(g) an ultrafiltration step prior to step (a), (b) and/or (e).

The content of different fractions can be determined by SDS-PAGE as an in-process control, and selected fractions combined or discarded as appropriate; see, e.g., Fig 2 for control of EPO elutes from the RP-HPLC step.

In a preferred embodiment the EPO purified in accordance with the method of the present invention is human recombinant EPO. Thus, the EPO molecule is preferably provided by inducing the expression of an EPO encoding gene in a host cell. A "host cell" is understood as an animal or human cell whose genome contains an active EPO gene and this EPO gene is transcribed and translated during culture of the cell in a serum-free medium. The EPO gene can be introduced into this host cell as an exogenous gene, preferably with regulation elements (see, e.g., European patent applications EP-B 0 148 605 and EP-B 0 209), can already be present in the host cell as an active endogenous gene or can become activated as an endogenous non-active gene. Such an activation of endogenous genes can be achieved by the specific introduction of regulation elements into the genome by homologous recombination, for example. International applications WO 91/09955 and WO 93/09222 describe examples of such methods.

Mammalian cells are usually used as host cells. If an exogenous human EPO gene is introduced, e.g. CHO or BHK cells can be used as host cells. If an endogenous EPO gene is used for the expression, it is expedient to use human cells such as kidney, liver or lymph cells. Preferably, the EPO is human recombinant EPO produced in CHO cells. The recombinant production of EPO in CHO is usually carried out with the addition of foetal calf serum and optionally bovine insulin in the culture medium. As a result, an EPO preparation produced in this manner bears a potential risk for infections with viruses or TSE-inducing agents as it may contain at least traces of such animal-derived agents even after purification. It is known that a serum-free fermentation of recombinant CHO cells which contain an EPO gene can be carried out using the methods of the state of the art; see, e.g., European patent applications EP 1 394 179, EP 0 513 738 and EP 0 267 678 and in a general form by Kawamoto et al., Analytical Biochem. 130 (1983) 445-453, Kowar and Franek,, Methods in Enzymology 421 (1986), 277-292, Bavister, Expcology 271 (1981), 45-51, European patent applications EP 0 248 656, EP 0 481 791, EP 0 307 247, EP 0 343 635 and international application WO 88/00967.

Derivatives and fragments of EPO, which have an analogous activity and are produced after culturing an EPO-producing host cell, can also be produced in a pure form by the processes according to the invention. The DNA and protein sequences of human EPO are described, for example, in European patent applications EP 0205 564 and EP 0 209 539.

In order to produce EPO the host cells containing the EPO gene can be adapted to medium which is free of proteins from natural sources by passaging in low volume cultures. The adapted cells are optionally cryopreserved, taken as required from an established cell bank and expanded in serum-free medium as described in, e.g., European patent application EP 1 394 179. For purification the cell-free culture supernatant of the host cell is preferably isolated and subjected to the purification process according to the present invention after filtration. Before carrying out the purification process, it is possible to carry out an additional filtration if necessary to separate turbidities or debris and/or to perform a concentration by ultrafiltration.

Also described herein is a preparation of glycosylated EPO isoforms purified by a method of the present invention as described above and preferably performed as illustrated in the Examples. Typically, the EPO is human recombinant EPO. Advantageously, the EPO preparation is substantially free of non-O-glycosylated EPO isoforms. The term "substantially free of non-O-glycosylated EPO isoforms" means that the EPO preparation typically contains less than 10% non-O-glycosylated EPO isoforms, preferably less than 5% and advantageously less than 1% non-O-glycosylated EPO isoforms. Put in other words, the EPO preparation typically contains at least 90% O-glycosylated EPO isoforms, preferably at least 95% and most preferably at least 99% O-glycosylated EPO isoforms by way of which the EPO preparation disclosed herein may be distinguished from EPO preparations purified according to methods known in the art; see, e.g., by SDS page as illustrated in Fig. 2 and Fig. 3. The main analytical techniques to characterize the glycosylation status are MALDI/TOF-MS and HPAEC-PAD. Other techniques to characterize the samples comprised SDS-PAGE, IEF, UV, CD, fluorescence spectroscopy and NMR. For example, determination of O-glycosylation of EPO preparations can be performed by a MALDI/TOF-MS analysis of tryptic peptides of the EPO molecule after separation of the peptide mixture through RP-HPLC C₄-phase according to the monograph of Eur. Pharm. While the non-glycosylated tryptic peptide containing the Ser-126 moiety has a mass of 1466.6, the corresponding peptide with a GalNAc moiety (Hex-NAc) should have a mass increment of 203 (m/z = 1669) and the Gal-GalNAc (Hex-NAc-Hex) derivative should have an increment mass of 365 corresponding to a mass of m/z = 1830. Corresponding experiments performed as described herein using MALDI/TOF-MS analysis confirmed the SDS-PAGE pattern of the EPO preparations after release of the N-glycans by polypeptide-N glycosidase (PNGase) in that substantially exclusively only O-glycosylated forms of the EPO protein could be detected, compared to about 15% of non-O glycosylated isoforms present in the standard EPO preparations.

In this context, the minimum value for specific EPO activity is typically 100,000 IU per mg (glycoprotein). In one embodiment, the EPO preparation has an activity of > 110,000 IU / mg which can be achieved due to the enrichment of highly sialylated EPO isoforms.

Thus, the EPO preparation described herein is particularly suited for therapeutic application. Accordingly, disclosed herein is a pharmaceutical composition comprising the EPO preparation as defined above. In this context, described herein is also a process for the manufacture of a pharmaceutical composition, the process comprising preparing and isolating EPO in the form of a glycoisoform mixture as defined above, and providing a mixture of the thus prepared and isolated EPO with a pharmaceutically acceptable carrier. In one embodiment, described herein is a stable pharmaceutical formulation of the EPO preparation containing tris-(hydroxymethyl)-aminomethane as stabilizer, whereby the formulation does not contain amino acids or human serum albumin, most preferably comprising as a pH buffering agent a sodium phosphate buffer, as stabilizer tris-(hydroxymethyl)-aminomethane in an amount of 10 to 200 mM and/or NaCl in an amount of 20-150 mM, and a pharmaceutical quantity of purified EPO. For further embodiments of the EPO formulation see European patent application EP 1 537 876.

Pharmaceutical compositions of the present invention are characterized as being at least sterile and pyrogen-free. As used herein, "pharmaceutical composition" includes formulations for human and veterinary use. Methods for preparing pharmaceutical compositions of the invention are within the skill in the art, for example as described in Remington's Pharmaceutical Science, 17th ed., Mack Publishing Company, Easton, Pa. (1985) and update version Remington: The Science and Practice of Pharmacy (2000) by the University of Sciences in Philadelphia, ISBN 0-683-306472.

These and other embodiments are disclosed and encompassed by the description and examples of the present invention. Further literature concerning any one of the materials, methods, uses and compounds to be employed in accordance with the present invention may be retrieved from public libraries and databases. For example, the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. There is no admission that any document cited is indeed prior art as to the present invention. A more complete understanding can be obtained by reference to the following specific examples. In particular, the examples relate to preferred embodiments of the present invention.

### EXAMPLES

Unless defined otherwise, all technical and scientific terms used herein have the same meaning as commonly understood by one of ordinary skill in the art (e.g., in cell culture, molecular genetics, nucleic acid chemistry, hybridization techniques, protein chemistry and biochemistry). Standard techniques are used for molecular, genetic and biochemical methods (see generally, Sambrook et al., Molecular Cloning: A Laboratory Manual, 2nd ed. (1989) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology) and chemical methods.

### Starting Material

Medium containing EPO protein is produced by a large-scale perfusion culture of a transformed Chinese Hamster Ovary cell line (CHO dhfr⁻) containing an amplified human EPO gene as described in the art; see, e.g., the literature cited supra. The concentration of EPO protein in the culture medium is related to the growth of the cells. In practice, the level of product expression (mg per volume) is limited by the maximum cell number achieved. Thus, production is maximized using a continuous perfusion culture system, which allows a high-density cell culture over an extended cultivation period. Perfusion is initiated at a defined cell density and the perfusion rate is maintained within a defined range manually adjusting the rate against cell number. Cell retention is achieved by standard procedures like acoustic settlers, filtration or centrifugation. Perfusate harvests are collected in portions at a refrigerated temperature. A total campaign typically consists of several weeks perfusion culture yielding 5-10 harvests. Residual cells and debris in the perfusates are removed from the perfusate harvest by depth filtration and the protein is preferably concentrated by tangential flow ultrafiltration with a molecular weight cut off of 30 kDa. Concentrates are split in aliquots and are stored at ≤ -70 °C until all harvests have been collected. Concentrated harvests are removed from the freezer and put in a Freeze-Thaw unit. Pooled and thawed harvests are clarified by filtration through a 0.45 µm depth filter. All downstream purification steps after thawing are conducted at ambient temperature (17 to 25°C).

### Composition of buffers and solutions

The buffer solutions for each purification step are described in Table 1 below.

**Table 1: Buffer compositions.**

| **Step Use** | **Buffer/Solution** | **Use** |
|---|---|---|
| Affinity Chromatography | 20 mM Tris.HCl, 1.5 M NaCl, pH 7.5 | Pre-equilibration buffer |
| | 20 mM Tris.HCl, pH 7.5 | Equilibration buffer |
| | 20 mM Tris.HCl, pH 7.5 | Washing buffer |
| | 20 mM Tris.HCl, 1.5 M NaCl, pH 7.5 | Elution buffer |
| | 5 M Urea | Regeneration |
| | 0.5 M NaOH | Sanitization |
| | 0.01 M NaOH | Storage |
| Diafiltration | 1 M NaOH | Sanitizing |
| | 20 mM Tris.HCl, pH 7.0 | Equilibration and diafiltration Buffer |
| Anion Exchange Chromatography | 20 mM Tris.HCl, 1.0 M NaCl, pH 7.0 | Pre-equilibration buffer |
| | 20 mM Tris.HCl, pH 7.0 | Equilibration and Washing Buffer |
| | 20 mM Tris.HCl, pH 7.0 | Elution Solution A |
| | 20 mM Tris.HCl, 0.5 M NaCl, pH 7.0 | Elution Solution B |
| | 20 mM Tris.HCl, 1.0 M NaCl, pH 7.0 | Regeneration buffer |
| | 1 M NaOH | Sanitizing |
| | 0.01 M NaOH | Storage |
| RP-HPLC Chromatography | 0.1 % (v/v) TFA | Equilibration |
| | 0.1 % (v/v) TFA in WFI | Elution Solution A |
| | 100% (v/v) Acetonitrile + 0.1 % (v/v) TFA | Elution Solution B |
| | 100% (v/v) acetonitrile | Regeneration |
| | 60% (v/v) acetonitrile | Cleaning and storage |
| Cation Exchange Chromatography | 100 mM Glycine.HCl, pH 2.0 | Pre-equilibration buffer |
| | 20 mM Glycine.HCl, pH 2.0 | Equilibration buffer |
| | 20 mM Glycine.HCl, pH 2.0 | Washing buffer |
| | 10 mM NaPO4, 0.15 M NaCl, pH 7.2 | Elution buffer |
| | 10 mM NaPO4, 1.0 M NaCl, pH 7.2 | Regeneration buffer |
| | 0.01 M NaOH | Storage |
| | 1 M NaOH | Sanitizing |
| Ultrafiltration | 1 M NaOH | Sanitizing |
| | 10 mM NaPO4, 0.15 M NaCl, pH 7.2 | Equilibration buffer |
| Size Exclusion Chromatography | 0.5 M NaOH | Sanitization |
| | 100 mM NaPO4, pH 7.2 | Pre-equilibration buffer |
| | 10 mM NaPO4, 0.15 M NaCl, pH 7.2 | Equilibration buffer |
| | 1.0 M NaOH | Sanitization |
| | 0.01 M NaOH | Storage |
| Nanofiltration | 10 mM NaPO4, 0.15 M NaCl, pH 7.2 | Equilibration buffer |

Five subsequent column chromatographic processes are utilized to purify each pooled concentrate to yield a batch of highly sialylated and O-glycosylated EPO in therapeutic grade; see also Fig. 1.

### Example 1: Capturing EPO and reduction of potential contaminants by affinity chromatography with Blue Sepharose 6FF

Blue Sepharose 6FF is an agarose resin covalently linked to the dye Cibacron Blue and is used to preferentially bind EPO in the presence of contaminants contained in the fermentation harvest. The product stream is first purified by affinity chromatography as specified in Table 2.

**Table 2: Parameters for performance of Blue Sepharose chromatography.**

| No. | Production procedure | Control | Desired value / Tolerance |
|---|---|---|---|
| **1.** | **Blue-Sepharose** | | |
| | - 10 x 12 cm = 1 L (BPG 100/500) | | |
| | - Installation: BioProcess | | |
| | - Application: 2.4 L concentrate = max. 3,000 mg EPO (1 - 1.5 mg EPO/ml) | | |
| | - Eluate: ∼ 500 ml pool in 100 ml Schott flasks (~ 4 mg EPO/ml) | | |
| 1.1 | Column loading and qualification | HETP / asymmetry | N > 2,500 / m 0.7 < Aₛ < 1.8 |
| 1.2 | Thawing of concentrates | Temperature | 20±32°C |
| | | Time | 2 h |
| 1.3 | Filtration of concentrates | Bulk filter / sterile filter Sartobran 300 capsule | From optimization run |
| 1.4 | Column sanitization | NaOH concentration | 0.1 N |
| | | Time | 1h,90cm/h |
| 1.5 | Performance run | Flow rate | 7 ± 0.5 L / h |
| | | Temperature | 22 ± 2°C |
| | | Loading | max. 3 mg EPO/ml gel |
| | | | max. 3,000 mg EPO |
| | | Step gradient | with 1.5 M NaCl |
| 1.6 | Pooling criteria | Initiation of pool | OD > 0.15 |
| | | Termination of pool | OD < 0.15, but Vol. ≤ 0.5 CV |
| 1.7 | Sample storage | Temperature Time | RT = 22 ± 2°C overnight, max. 18 h |
| 1.8 | Column post-processing | 1. Urea: Concentration Time | 5M 30 min, 90cm / min. |
| | | 2. NaOH: Concentration time | 0.1 N 1 h, 90 cm / min. |
| Duration of run: | | About 5 h | |

The column is packed with Blue Sepharose 6FF resin. Following packing, the column is qualified for theoretical plates and asymmetry factor. The column is sanitized with 0.5 M NaOH for 1 hour and then rinsed with Water for Injection (WFI). Prior to loading, the column is equilibrated with 20 mM Tris.HCl, 1.5 M NaCl, pH 7.5, followed by 20 mM Tris.HCl, pH 7.5. The sample is loaded onto the column and the column is washed with of 20 mM Tris.HCl, pH 7.5. The sample is eluted using 20 mM Tris.HCl, 1.5 M NaCl, pH 7.5. Sample collection is initiated after the minimum OD between the pre-peak and the main peak is not less than 0.15. Collection is terminated when the OD is equal to or less than 0.15. The eluate is collected into a sterile disposable bag.

After elution, the column is rinsed with WFI and is then stripped by washing with 5 M urea. Following further washes with WFI, the column is sanitized with 0.5 M NaOH. The column is rinsed with WFI and is stored in 0.01 M NaOH.

Further information on the chromatographic resin is provided in the manufacturer's regulatory support file (Representative GE Healthcare Blue Sepharose Regulatory Support File).

### Example 2: Concentration of EPO by diafiltration

The eluate is concentrated by ultrafiltration and diafiltered using a 10 kDa cut-off membrane on a tangential flow filtration unit; see Table 3.

**Table 3: Parameters for performance of diafiltration.**

| No. | Production procedure | Control | Desired value / Tolerance |
|---|---|---|---|
| **2.** | **Diafiltration** | | |
| | - Installation: Proflux | | |
| | - 0.1 m² Hydrosart 10 kDa membrane | | |
| | - 500 ml BS eluate, concentrate to 300 ml, diafiltrate with 6-fold volume, rinse with 2 x 200 ml = 700 ml retentate | | |
| 2.1 | Membrane qualification | Water equivalent | |
| 2.2 | Membrane sanitization | Reagent | 1 N NaOH |
| | | Time | at least 30 min. |
| 2.3 | Performance diafiltration | Retentate flow | |
| | | Temperature | 22 ± 2°C |
| | | Inlet pressure | 1 bar |
| | | Outlet pressure | 0.5 bar |
| | | Diafiltration volume | 6-fold |
| 2.4 | Concentrate storage | Temperature | 4 ± 2°C |
| | | Time | direct re-use, max. 24 h |
| 2.5 | Membrane | Reagent | 1 N NaOH |
| | post-processing | Time | at least 30 min. |
| 2.6 | IPC release | Conductivity | < 2.5 mS / cm |
| Duration of diafiltration: | | About 2 h + 2 h pre- and post-processing | |

A normalized water permeability test (NWP) is performed before using the filter unit. The filter unit is sanitized with 1 M NaOH for at least 1 hour and is then washed with WFI. Following washing, the filter unit is equilibrated using 20 mM Tris-HCl, pH 7.0. The sample is then loaded and filtered at a transmembrane pressure of not more than 1 bar. Diafiltration is terminated when the conductivity of the permeate is less than 2.5 mS/cm.

### Example 3: Enrichment of acidic isoforms of EPO and further removal of contaminants via anion exchange chromatography with Q-Sepharose HP

Anion exchange chromatography with Q-Sepharose HP resin is used for the enrichment of acidic isoforms of EPO, the further removal of contaminants (e.g. DNA, HCP) and the elimination of any dye ligand that may have leached from the first column. In addition, the anion exchange chromatography is an effective step for removal of adventitious viruses. Thus, the diafiltrate is processed by anion exchange chromatography, as specified in Table 4, followed by 0.2µm filtration of Q eluate fractions and fraction pool.

**Table 4: Parameters for performance anion exchange chromatography.**

| No. | Production procedure | Control | Desired value / Tolerance |
|---|---|---|---|
| **3.** | **Q-Sepharose High Performance** | | |
| | - 6.2 x 16.5 cm = 500 ml (Vantage VA 60 x 500) | | |
| | - Installation: ÄKTA Purifier | | |
| | - Application: 700 ml diafiltrate = max. 1,500 mg EPO | | |
| | - Eluate: About 2,000 ml in 250 ml Schott flasks: 100 - 200 ml fractions | | |
| 3.1 | Column loading and qualification | HETP / asymmetry | N > 4,000 / m 0.6 < Aₛ < 2.0 |
| 3.2 | Thawing of concentrates | NaOH concentration | 1±0.05N |
| | | Time | 1h,90cm/h |
| 3.3 | Filtration of concentrates | pH after equilibration | 7.0 ± 0.1 |
| | | Flow rate | 45 ± 3 ml / min. = 90 cm/h |
| | | Temperature | 22 ± 2°C |
| | | Loading | max. 3 mg EPO/ml gel |
| | | | max. 1,500 mg EPO |
| | | Gradient | 0 - 25 M NaCl in 30 CV |
| 3.4 | Column sanitization | Initiation of pool | OD ∼ 85% of peak max., |
| | | Termination of pool | OD ∼ 25% of peak max., |
| | | | For both proteins / EPO |
| 3.5 | Performance run | Temperature | RT = 22 ± 2°C |
| | | Time | Overnight, max. 18 h |
| 3.6 | Pooling criteria | NaOH concentration | 1 ± 0.05 N |
| | | Time | 1 h,90cm/h |
| 3.7 | Sample storage | Protein / EPO | < 1.5 |
| | | to be determined | After optimization run |
| Duration of run: | | About 6 h | |

The column is packed with Q-Sepharose HP resin. Following packing, the column is qualified for theoretical plates and asymmetry factor. The column is rinsed with WFI and is then sanitized with 1 M NaOH for 1 hour. Following sanitization, the column is rinsed with WFI. Prior to loading, the column is equilibrated with 20 mM Tris.HCl, 1.0 M NaCl, pH 7.0, followed by 20 mM Tris.HCl, pH 7.0. The sample is loaded onto the column and the column is washed with 20 mM Tris.HCl, pH 7.0. The sample is eluted with a linear gradient using 20 mM Tris.HCl, 0.5 M NaCl, pH 7.0. EPO is collected as fractions through the linear gradient. The core fraction is initiated at about 85% to 95% Peak Max of the decreasing slope (UV) and is terminated when the UV value has decreased to about 25% Peak Max.

Each anion exchange chromatography eluate fraction is filtered using a 0.2µm filter unit. The fractions are held at 22 ± 3°C before pooling for up to 20 hours (Hold Step I) in sterile disposable bags while in-process testing is being conducted. Selected fractions have to be combined in order to achieve the desired isoform distribution specification. In order to determine which fractions will be pooled, small-scale samples of the fractions are prepared and analyzed by capillary zone electrophoresis (CZE). The combination of fractions that comes closest to the desired isoform distribution profile is preferably chosen and these fractions are pooled for further purification.

Between uses, the resin is regenerated by rinsing with 20 mM Tris.HCl, 1 M NaCl, pH 7.0, followed by a wash with WFI. The column is then sanitized with 1 M NaOH for 1 hour and washed with WFI. The column is stored in 0.01 M NaOH.

Further information on the chromatographic resin is provided in the manufacturers regulatory support file (Representative GE Healthcare Q-Sepharose Regulatory Support File).

### Example 4: Depletion of EPO molecules that are not glycosylated at the Ser126 residue and removal of further contaminants by RP-HPLC with C4 resin

RP-HPLC, using C4 resin separates EPO from potential contaminants on the basis of hydrophobicity and, if desired, can also decrease the amount of EPO molecules that are not glycosylated at the Ser126 residue. In addition this step eliminates any remaining dye ligand that may have leached from the first column. The filtered anion exchange chromatography fraction pool is purified by reverse phase chromatography, as specified in Table 5.

**Table 5: Parameters for performance of RP-HPLC.**

| No. | Production procedure | Control | Desired value / Tolerance |
|---|---|---|---|
| **4.** | **Reverse Phase Column** | | |
| | - 5 x 25 cm = 491 ml Vydac C₄ Reverse Phase Column (10 - 15 µm) | | |
| | - Installation: Waters Deltaprep 4000 | | |
| | - Application: 1,600 - 2,400 ml eluate of Q Sepharose = max. 1,570 mg EPO | | |
| | - Eluate: About 500 ml in 50 ml Falcon tubes (~ 2 mg EPO / ml) | | |
| 4.1 | Column qualification | HETP / asymmetry | Separation |
| | | | Standard mixture |
| 4.2 | Column sanitization | - | - |
| 4.3 | Performance run | pH after equilibration | 2.0 ± 0.25 |
| | | Flow rate | 100 ± 5 ml / min. = 300 cam / h |
| | | Temperature | 22 ± 2°C |
| | | Loading | max. 3.2 mg EPO/ml gel |
| | | | max. 1,570 mg EPO |
| | | Gradient | 50 - 80 % buffer B (70% acetonitrile) |
| 4.4 | Pooling criteria | Initiation of pool | OD₂₈₀ >0.01 |
| | | Termination of pool | ~ 45% of peak max. |
| 4.5 | Sample storage | Temperature | RT = 22 ± 2°C |
| | | Time | Overnight, max. 18 h |
| 4.6 | Column post-processing | Acetonitrile | 100 % (V/V) |
| | | Time | 1 h |
| Duration of run: | | About 6 h | |

The column is packed with C4 resin. The column is equilibrated with 0.1% (v/v) TFA in WFI. Prior to use, the column performance is defined by determining the plate number and asymmetry factor. The sample is loaded onto the column and is then eluted using the linear gradient described in Table 1 and 6. The eluate is collected once the absorption at 280 nm has reached 0.01 AU and is terminated when the absorption has decreased to 40% to 45% of the peak maximum on the decreasing slope. The eluate is collected into a glass bottle.

As mentioned the gradient is formed by water / TFA 0.1% (solvent A) and water 30% / acetonitrile 70% (v/v) / TFA 0.1 % (solvent B). In particular, a pool of thawed fractions of the Q Sepharose HP eluate was used as sample and filtered through a 0.2 µm filter. After rinsing the column with two column volumes (CV) of Milli-Q water and equilibration with 4 CV solvent A the sample was loaded on the column and the gradient was applied according to Table 6.

**Table: 6: Gradient for performance of the RP-HPLC.**

| Time (min.) | %A | %B |
|---|---|---|
| 0 | 100 | 0 |
| 9.2 | 50 | 50 |
| 18.3 | 50 | 50 |
| 52.7 | 20 | 80 |
| 59.5 | 0 | 100 |
| 75.6 | 0 | 100 |
| 91.6 | 100 | 0 |
| 108.8 | 100 | 0 |
| Total volume (L) | ∼5 | ∼6 |

The eluate is collected in 50 ml fractions which are stored at -20°C.

Between uses, the resin is regenerated and the column is qualified by measuring the theoretical plates and the asymmetry factor. Initially, the column is washed using a gradient of 70% (v/v) acetonitrile to 100% (v/v) acetonitrile. The column is then washed with 100% (v/v) acetonitrile. The acetonitrile concentration is reduced by washing with a gradient of 100% (v/v) acetonitrile to 60% (v/v) acetonitrile and the column is then stored in 60% (v/v) acetonitrile.

Further information on the chromatographic resin is provided in the manufacturer's regulatory support file (Representative Vydac C4 Resin Regulatory Support File).

### Example 5: Virus inactivation by incubation of EPO in acetonitrile / TFA

Following the RP-HPLC step, the eluate is held for 60 to 180 minutes at 22 ± 3°C. The concentration of the acetonitrile in the eluate is approximately 41% (v/v) and the concentration of TFA is approximately 0.1% (v/v). During this stage of the process, the hold temperature and the holding time are controlled.

### Example 6: Removal of RP-HPLC solvents and aggregated EPO species via cation exchange chromatography with MacroPrep High S

Cation exchange chromatography with MacroPrep High S resin is used to remove RP-HPLC solvents and aggregated species and to exchange the buffer in rather short time. The RP-HPLC eluate is processed by cation exchange chromatography, as specified in Table 7, followed by 0.2µm filtration of MacroPrep eluate.

**Table 7: Parameter for performing cation exchange chromatography.**

| No. | Production procedure | Control | Desired value / Tolerance |
|---|---|---|---|
| **5.** | **Macroprep HighS** | | |
| | - 5 x 12.5 cm = 250 ml | | |
| | - Installation: ÄKTA Purifier | | |
| | - Application: 400 - 500 ml eluate of the RP column = max. 1,250 mg EPO | | |
| | - Eluate: ∼ 250 ml in 50 ml Falcon tubes (∼ 3 mg EPO / ml) | | |
| 5.1 | Column loading and qualification | HETP / asymmetry | N > 2,500 / m |
| | | | 0.6 < As < 1.8 |
| 5.2 | Column sanitization | NaOH Concentration | 1 ± 0.05 N |
| | | Time | 1 h |
| 5.3 | Performance run | pH after equilibration | 2.0 ± 0.2 |
| | | Flow rate | 50 ± 3 ml / min. = 150cm/h |
| | | Temperature | 22 ± 2°C |
| | | Loading | max. 5 mg EPO/ml gel max. 1,250 mg EPO |
| | | Gradient | Step gradient with pH and NaCl after optimization run |
| 5.4 | Pooling criteria | Initiation of pool | OD₂₈₀ > 0.03 |
| | | Termination of pool | OD₂₈₀ < 0.03 |
| 5.5 | Sample storage | Temperature | RT = 22 ± 2°C |
| | | Time | concentrate immediately after run, max. 24 h |
| 5.6 | Column post-processing | NaOH concentration | 1 ± 0.05 N |
| | | Time | 1 h |
| Duration of run: | | About 3 h + 3 h pre- and post-processing | |

The column is packed with MacroPrep High S resin. Following packing, the column is qualified for theoretical plates and asymmetry factor. The column is rinsed with WFI and then sanitized with 1 M NaOH for at least 1 hour. Following sanitization, the column is rinsed with WFI. Prior to loading, the column is preequilibrated with 100 mM Glycine.HCl, pH 2.0, and is then equilibrated with 20 mM Glycine-HCl, pH 2.0. The sample is loaded onto the column and the column is washed with 20 mM Glycine-HCl, pH 2.0. The sample is eluted using the steep gradient described in Table 1. The eluate is collected once the absorption at 280 nm has reached 0.03 AU and is terminated when the absorption has reached 0.03 AU. The eluate is stored in sterile disposable bags. The cation exchange chromatography eluate is filtered using a 0.2 µm filter unit prior to further purification and/or starting the hold time at 22 ± 3°C for up to 20 hours in a sterile disposable bag.

Between uses, the resin is regenerated. The column is washed with 10 mM NaPO₄ 1.0 M NaCl, pH 7.2 and is then rinsed with WFI. The EPO aggregates elute in the regenerate. The column is sanitized with 1 M NaOH for at least 1 hour and rinsed with WFI. The column is stored in 0.01 M NaOH.

Further information on the resin is supplied in the manufacturer's regulatory support file (Representative BioRad MacroPrep High S Regulatory Support File.

### Example 7: Ultrafiltration

Optionally, the filtered cation exchange chromatography eluate is further concentrated by ultrafiltration using a tangential flow filtration unit with a 10 kDa cut-off to achieve the desired volume reduction. Information on the filtration step is provided in Table 8.

**Table 8: Parameters for performing ultrafiltration.**

| No. | Production procedure | Control | Desired value / Tolerance |
|---|---|---|---|
| **6.** | **Ultrafiltration** | | |
| | - Installation: Amicon Stirred Cell | | |
| | - 76 mm Flat Membrane YM-10 kDa | | |
| | - concentrate 250 ml to 80 ml, rinse with 2 x 10 ml = 100 ml | | |
| 6.1 | Membrane qualification | Water equivalent | |
| 6.2 | Membrane sanitization | Reagent | 0.1 N NaOH |
| | | Time | at least 1 h |
| 6.3 | Performance | Temperature | 22 ± 2°C |
| | ultrafiltration | Inlet pressure | 1 bar |
| | | UV₂₈₀ in the retentate | |
| | | UV₂₈₀ in the filtrate | < 0.05 |
| 6.4 | Concentrate storage | Temperature | RT = 22 ± 22°C |
| | | Time | Overnight, max. 18 h |
| 6.5 | Membrane | Reagent | 0.1 N NaOH |
| | post-processing | Time | at least 1 h |
| 6.6 | IPC release | EPO concentration | 5 < X <7 mg / ml |
| | | Volume retentate | ≤ 100 ml |
| Duration of diafiltration: | | About 1 h + 2 h pre- and post-processing | |

The filter unit is sanitized with 1 M NaOH and is then washed with WFI. Following washing, the filter unit is equilibrated using 10 mM NaPO₄ 0.15 M NaCl, pH 7.2 buffer. The sample is then loaded and filtered at 22 ± 3°C at a feed pressure of 0.7 to 1.1 bar. The retentate is collected into a sterile disposable bag.

### Example 8: Removal of any remaining EPO aggregates and other contaminants via size exclusion chromatography with Superdex S200 Prep Grade

Superdex S200 prep grade size exclusion chromatography is the final (polishing) chromatography step and is employed to remove any possible remaining aggregates and to formulate the drug substance bulk solution. The concentrate after ultrafiltration (Example 7) or the eluate after CEX chromatography (Example 6) is processed by size exclusion chromatography, as specified in Table 9, followed by 0.2µm filtration of SE-HPLC eluate as described in Example 9.

**Table 9: Parameters for performing size exclusion chromatograph.**

| No. | Production procedure | Control | Desired value / Tolerance |
|---|---|---|---|
| **7.** | **Superdex 200 pg** | | |
| | - 10 x 70 cm = 5.5 L (BPG 100 / 950) | | |
| | - Installation: ÄKT A Purifier | | |
| | - Application: 100 ml Concentrate of the UF | | |
| | - Eluate: ~ 300 ml in 50 ml Falcon tubes (~ 1.5 mg EPO / ml) | | |
| 7.1 | Column loading and qualification | HETP / asymmetry | N > 5,000 / m |
| | | | 0.7 < Aₛ < 1.8 |
| 7.2 | Column sanitization | NaOH concentration | 0.5 ± 0.05 N |
| | | Time | at least 1 h |
| 7.3 | Performance run | pH after equilibration | 7.2 ± 0.2 |
| | | Flow rate | 32 ± 2 ml/min. = 24 cm/h |
| | | Temperature | 22 ± 2°C |
| | | EPO concentration in the application | 5 < X < 7 mg EPO/ml |
| | | Vol. appl. X% v CV | max. 4% |
| 7.4 | Pooling criteria | Initiation of pool | OD₂₈₀ > 0.01 |
| | | Termination of pool | OD₂₈₀ < 0.01, max. 300 ml |
| 7.5 | Pool storage | Temperature | is "nanofiltered" on the same day |
| | | Time | |
| 7.6 | Column post-processing | NaOH concentration | 0.5 ± 0.05 N |
| | | Time | at least 1 h |
| 7.7 | IPC release | EPO concentration | 1 < X < 3 mg / ml |
| Duration of run: | | About 3 h + 12 h pre- and post-processing | |

The column is packed with Superdex S200 prep grade resin. Following packing, the column is qualified for theoretical plates and asymmetry factor. The column is sanitized with 0.5 M NaOH for 1 hour and is then rinsed with WFI. Prior to loading, the column is pre-equilibrated with 100 mM NaPO₄ pH 7.2, and is then equilibrated with 10 mM NaPO₄ 0.15 M NaCl, pH 7.2. The sample is loaded onto the column and the eluate is collected once the absorption at 280 nm has increased over 0.01 AU and is terminated when the absorption has reached 0.01 AU. The eluate is collected in sterile disposable bags. The size exclusion chromatography eluate is filtered in a sterile disposable bag prior to further processing using a 0.2 µm filter. After use, the column is washed with WFI. The column is then sanitized using 0.5-1 M NaOH (0.6 CV) for 1 hour and is stored in 0.01 M NaOH.

Further information on the resin is provided in the manufacturer's regulatory support file (Representative GE Healthcare Superdex Regulatory Support File).

### Example 9: Virus removal from the EPO preparation via nanofiltration

Finally a 15 nm nanofiltration step is included in the purification process to increase the virus safety of the drug substance. The size exclusion chromatography eluate filtrate is nanofiltered using a Planova 15N filter, which serves to remove viral adventitious agents from the eluate. The nanofilter unit is prepared by flushing with 150 ml 10 mM NaPO₄ 0.15 M NaCl, pH 7.2. The 0.2 µm filtered size exclusion chromatography eluate is pumped through the nanofilter and the filtrate is pooled into a sterile disposable bag.

### Example 10: Filling, storage and transportation

The filling process is the final step of the EPO drug substance manufacturing process. The final containers are sterilized and depyrogenated prior to filling. The nanofiltered fraction is dispensed, using a peristaltic pump, into bulk drug substance storage containers of a size of 30 ml volumes. This operation is performed in a laminar flow hood (local protection) in a filling room at ambient temperature. Storage of final product can be done at 70°C, for example in Teflon-coated PP tubes. The drug substance can be transferred to the drug product manufacturing location and the temperature of the bulk drug substance is maintained during transport at less than -70°C using dry ice.

## Claims

1. A method of purifying biologically active O-glycosylated at serine 126 erythropoietin (EPO) isoforms from a complex protein mixture in pure form suitable as a drug substance, wherein the method comprises:
(a) a dye affinity chromatography step for capturing and concentrating the EPO containing solution and providing the major reduction of potential contaminants;
(b) an anion exchange chromatography step for the enrichment of acidic isoforms of EPO, further removal of contaminants and the elimination of any dye ligand that may have leached from the first column;
(c) a reverse phase high performance liquid chromatography (RP-HPLC) step under conditions in order to remove EPO molecules that are not glycosylated at the Ser126 residue and to remove contaminants;
(d) a cation exchange chromatography step for removing RP-HPLC solvents and aggregated species, to exchange the buffer, and to concentrate the EPO fraction; and
(e) a size exclusion chromatography step as the final chromatography step employed to remove any possible remaining aggregates and other contaminants.

2. The method of claim 1, wherein the elution of EPO in one or more of the chromatography steps is carried out by a step or gradient elution.

3. The method of claim 1 or 2, wherein
(a) the affinity chromatography step is performed with a dye chromatography resin;
(b) the anion exchange chromatography step is performed with anion exchange resins or membranes that contain Diethylaminoethyl-groups (DEAE), quaternary aminoethyl-groups (QAE), quaternary ammonium-groups (Q), Dimethylaminoethyl-groups (DMAE) and/or Trimethylaminoethyl-groups (TMAE) as functional groups;
(c) the RP-HPLC is performed with resins that contain Methyl-, Butyl-, Phenyl-, Propyl- and/or Octyl- groups as functional groups;
(d) the cation exchange chromatography step is performed with resins that contain sulfopropyl cation exchange material; and/or
(e) the size exclusion chromatography step is performed with a gel filtration medium selected from the group of Superdex, Sephacryl, Sephadex, Sepharose, Fractogel, Toyopearl, and Bio-Gel.

4. The method of claim 3, wherein
(a) the affinity chromatography step is performed with commercially available Blue-Sepharose;
(b) the anion exchange chromatography step is performed with commercially available Q-Sepharose;
(c) the RP-HPLC is performed with a commercially available C4 reversed-phase chromatography material;
(d) the cation exchange chromatography step is performed with commercially available Macro-Prep High S; and/or
(e) the size exclusion chromatography step is performed with commercially available Superdex-S200.

5. The method of any one of claims 1 to 4, wherein the anion exchange chromatography is used to select EPO isoforms, preferably using a linear salt gradient from 0 to 200mM NaCl in a buffer comprising 20mM Tris-HCl at a pH of about 7,0.

6. The method of any one of claims 1 to 5, wherein in the RP-HPLC EPO is eluted with a linear gradient of an organic solvent, preferably wherein a linear gradient is used from 0 to 70% acetonitrile in water and the solvents contain about 0,1% TFA and/or an isocratic elution of EPO with a solvent containing acetonitrile and about 0,1% TFA in water is used.

7. The method of any one of claims 1 to 6, which comprises prior to one or more of the chromatography steps an ultrafiltration step, and optionally a nanofiltration step as the final step.

8. The method of any one of claims 1 to 7 further comprising the following steps
(f) a nanofiltration step; and
(g) an ultrafiltration step prior to step (a), (b) and/or (e).

9. The method of any one of claims 1 to 8, wherein the EPO is human recombinant EPO, preferably wherein the EPO is human recombinant EPO produced in CHO cells.

## Patentansprüche

1. Verfahren zur Reinigung von biologisch aktiven O-glykosilierten an Serin 126 Erythropoietin (EPO) Isoformen aus einer komplexen Proteinmischung in reiner Form geeignet als Arzneimittel, wobei das Verfahren umfasst:
(a) einen Farbaffinitätschromatographie-Schritt, um die EPO beinhaltende Lösung festzuhalten und zu konzentrieren und um eine größere Reduktion von potenziellen Verunreinigungen bereitzustellen;
(b) einen Anionenaustauschchromatographie-Schritt für die Anreicherung von sauren Isoformen von EPO, weitere Beseitigung von Verunreinigungen und die Eliminierung von jedem Farbligand der von der ersten Säule ausgewaschen worden ist;
(c) einen Umkehrphasenhochleistungsflüssigkeitschromatographie (RP-HPLC) - Schritt unter Bedingungen, um EPO Moleküle, die nicht glykosiliert sind am Ser126 Rest, zu beseitigen und um Verunreinigungen zu beseitigen;
(d) einen Kationenaustauschchromatographie-Schritt, um RP-HPLC Lösemittel und aggregierte Spezies zu beseitigen, um den Puffer auszutauschen und um die EPO Fraktion zu konzentrieren; und
(e) einen Größenausschlusschromatographie-Schritt als den letzten Chromatographie-Schritt, angewendet um alle möglichen verbleibenden Aggregate und andere Verunreinigungen zu beseitigen.

2. Verfahren nach Anspruch 1, wobei die Elution von EPO in einem oder mehreren Chromatographie-Schritten durchgeführt wird durch schrittweise oder Gradienten-Elution.

3. Verfahren nach Anspruch 1 oder 2, wobei
(a) der Affinitätschromatographie-Schritt mit einem Farbchromatographieharz durchgeführt wird;
(b) der Anionenaustauschchromatographie-Schritt mit einem Anionenaustauschharz oder Membranen, die Diethylaminoethyl-Gruppen (DEAE), quartäre Aminoethyl-Gruppen (QAE), quartäre Ammonium-Gruppen (Q), Dimethylaminoethyl-Gruppen (DMAE) und/oder Trimethylaminoethyl-Gruppen (TMAE) als funktionelle Gruppen beinhalten durchgeführt wird;
(c) die RP-HPLC mit Harzen, die Methyl-, Butyl-, Phenyl-, Propyl- und/oder Octyl-Gruppen als funktionelle Gruppen beinhalten durchgeführt wird;
(d) der Kationenaustauschchromatographie-Schritt mit Harzen, die Sulfopropyl-Kationenaustausch-Material beinhalten durchgeführt wird; und/oder
(e) der Größenausschlusschromatographie-Schritt mit einem Gelfiltrationsmedium ausgewählt aus der Gruppe von Superdex, Sephacryl, Sephadex, Sepharose, Fractogel, Toyopearl und Bio-Gel durchgeführt wird.

4. Verfahren nach Anspruch 3, wobei
(a) der Affinitätschromatographie-Schritt mit kommerziell erhältlicher Blue-Sepharose durchgeführt wird;
(b) der Anionenaustauschchromatographie-Schritt mit kommerziell erhältlicher Q-Sepharose durchgeführt wird;
(c) die RP-HPLC mit einem kommerziell erhältlichem C4 Umkehrphasenchromatographie-Material durchgeführt wird;
(d) der Kationenaustauschchromatographie-Schritt mit kommerziell erhältlichem Macro-Prep High S durchgeführt wird; und/oder
(e) der Größenausschlusschromatographie-Schritt mit kommerziell erhältlichem Superdex-S200 durchgeführt wird.

5. Verfahren nach einem der Ansprüche 1 bis 4, wobei die Anionenaustauschchromatographie verwendet wird, um EPO Isoformen zu selektieren, bevorzugt unter Verwendung eines linearen Salzgradienten von 0 bis 200mM NaCl in einem Puffer umfassend 20mM Tris-HCl bei einem pH von ungefähr 7,0.

6. Verfahren nach einem der Ansprüche 1 bis 5, wobei in der RP-HPLC EPO mit einem linearen Gradienten eines organischen Lösemittels eluiert wird, bevorzugt wobei ein linearer Gradient von 0 bis 70% Acetonitril in Wasser verwendet wird und die Lösemittel ungefähr 0,1% TFA beinhalten und/oder eine isokratische Elution von EPO mit einem Lösemittel beinhaltend Acetonitril und ungefähr 0,1% TFA in Wasser verwendet wird.

7. Verfahren nach einem der Ansprüche 1 bis 6, das vorausgehend zu einem oder mehreren der Chromatographie-Schritte einen Ultrafiltrations-Schritt und wahlweise einen Nanofiltrations-Schritt als den letzten Schritt umfasst.

8. Verfahren nach einem der Ansprüche 1 bis 7 ferner umfassend die folgenden Schritte
(f) einen Nanofiltrations-Schritt; und
(g) einen Ultrafiltrations-Schritt vorausgehend zu Schritt (a), (b) und/oder (e).

9. Verfahren nach einem der Ansprüche 1 bis 8, wobei das EPO humanes rekombinantes EPO ist, bevorzugt wobei das EPO humanes rekombinantes EPO hergestellt in CHO Zellen ist.

## Revendications

1. Procédé de purification d'isoformes d'érythropoïétine (EPO) O-glycolysée au niveau de la sérine 126, biologiquement actives à partir d'un mélange complexe de protéines sous forme pure convenant comme substance médicamenteuse, dans lequel le procédé comprend :
(a) une étape de chromatographie d'affinité de colorant pour capturer et concentrer la solution contenant l'EPO et pour fournir une réduction majeure des contaminants potentiels;
(b) une étape de chromatographie d'échange d'anions pour l'enrichissement des isoformes acides de l'EPO, l'élimination plus poussée des contaminants et l'élimination de tout ligand de colorant qui peut avoir lixivié à partir de la première colonne;
(c) une étape de chromatographie liquide haute performance à phase inversée (CLHP-PI) dans des conditions permettant de séparer les molécules d'EPO qui ne sont pas glycosylées au niveau du résidu ser126 et pour éliminer des contaminants ;
(d) une étape de chromatographie d'échange de cations pour éliminer les solvants de la CLHP-PI et les espèces agrégées, pour échanger le tampon et pour concentrer la fraction d'EPO ; et
(e) une étape de chromatographie d'exclusion de taille en tant qu'étape de chromatographie finale employée pour éliminer tout agrégat et tout autre contaminant restants possibles.

2. Procédé selon la revendication 1, dans lequel l'élution de l'EPO dans une ou plusieurs des étapes de chromatographie est réalisée par élution étagée ou à gradient.

3. Procédé selon la revendication 1 ou 2, dans lequel
(a) l'étape de chromatographie d'affinité est réalisée avec une résine de chromatographie à base d'un colorant;
(b) l'étape de chromatographie d'échange d'anions est réalisée avec des résines ou des membranes échangeuses d'anions qui contiennent des groupes diéthylaminoéthyle (DEAE), des groupes aminoéthyle quaternaire (QAE), des groupes ammonium quaternaire (Q), des groupes diméthylaminoéthyle (DMAE) et/ou des groupes triméthylaminoéthyle (TMAE) en tant que groupes fonctionnels;
(c) la CLHP-PI est réalisée avec des résines qui contiennent des groupes méthyle, butyle, phényle, propyle et/ou octyle en tant que groupes fonctionnels;
(d) l'étape de chromatographie d'échange de cations est réalisée avec des résines qui contiennent une matière échangeuse de cations de type sulfopropyle; et/ou
(e) l'étape de chromatographie d'exclusion de taille est réalisée avec un milieu de filtration sur gel choisi dans le groupe de Superdex, Sephacryl, Sephadex, Sepharose, Fractogel, Toyopearl et Bio-Gel.

4. Procédé selon la revendication 3, dans lequel
(a) l'étape de chromatographie d'affinité est réalisée avec du Blue-Sepharose disponible dans le commerce ;
(b) l'étape de chromatographie d'échange d'anions est réalisée avec du Q-Sepharose disponible dans le commerce ;
(c) la CLHP-PI est réalisée avec une matière de chromatographie à phase inversée C4 disponible dans le commerce;
(d) l'étape de chromatographie d'échange de cations est réalisée avec du Macro-Prep High S disponible dans le commerce ; et/ou
(e) l'étape de chromatographie d'exclusion de taille est réalisée avec du Superdex-S200 disponible dans le commerce.

5. Procédé selon l'une quelconque des revendications 1 à 4, dans lequel la chromatographie d'échange d'anions est utilisée pour choisir des isoformes d'EPO, de préférence avec un gradient linéaire de sel de 0 à 200 mM de NaCl dans un tampon comprenant 20 mM de Tris-HCl à un pH d'environ 7,0.

6. Procédé selon l'une quelconque des revendications 1 à 5, dans lequel, dans la CLHP-PI, l'EPO est éluée avec un gradient linéaire d'un solvant organique, de préférence dans lequel un gradient linéaire de 0 à 70 % d'acétonitrile dans de l'eau est utilisé et les solvants contiennent environ 0,1 % de TFA et/ou une élution isocratique d'EPO avec un solvant contenant de l'acétonitrile et environ 0,1 % de TFA dans de l'eau est utilisée.

7. Procédé selon l'une quelconque des revendications 1 à 6, qui comprend, avant une ou plusieurs des étapes de chromatographie, une étape d'ultrafiltration et éventuellement une étape de nanofiltration en tant qu'étape finale.

8. Procédé selon l'une quelconque des revendications 1 à 7 comprenant en outre les étapes suivantes :
(f) une étape de nanofiltration; et
(g) une étape d'ultrafiltration avant les étapes (a), (b) et/ou (e).

9. Procédé selon l'une quelconque des revendications 1 à 8, dans lequel l'EPO est de l'EPO recombinante humaine, de préférence dans lequel l'EPO est une EPO recombinante humaine produite dans des cellules CHO.
